# EUROPEAN PATENT APPLICATION

(11) **EP 4 471 004 A1**
(43) Date of publication of application: **04.12.2024**
(21) Application number: 22924219.3
(22) Date of filing: 27.12.2022
(51) Int. Cl.: C07C 231/10, C07C 237/06

(54) **METHOD FOR PRODUCING AMIDE COMPOUND**

(30) Priority: 28.01.2022 JP 2022011981
(71) Applicant: Nippon Soda Co., Ltd., Tokyo 100-7010 (JP)
(72) Inventor: NODA Kaoru, Takaoka-shi, Toyama 933-8507 (JP); TAKEHISA Katsuma, Takaoka-shi, Toyama 933-8507 (JP)
(74) Representative: Mewburn Ellis LLP
(86) International application number: PCT/JP2022/048272
(87) International publication number: WO 2023/145372

(57) **Abstract**

The present invention provides a method for producing a compound represented by formula (3) and/or a salt thereof such as 2-amino-2-methyl-N-(2,2,2-trifluoroethyl)propanamide, comprising chemically reacting a compound represented by formula (1) such as 4,4-dimethyl-1,3-oxazolidine-2,5-dione with a compound represented by formula (2) and/or a salt thereof such as 2,2,2-trifluoroethylamine, in which in formula (1), R¹ and R² each independently represents a hydrogen atom or a C1-6 alkyl group, in formula (2), R^{f} is a fluorine atom-containing C1-6 alkyl group, in formula (3), R¹ and R² are the same as those in formula (1), and R^{f} is the same as that in formula (2).

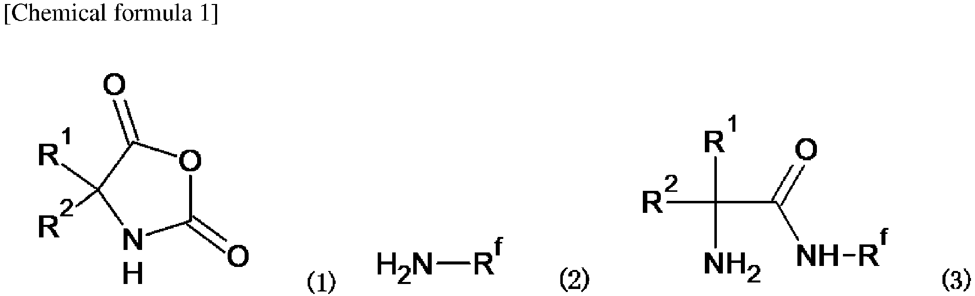

## Description

### [Technical Field]

The present invention relates to a method for producing an amide compound, and more specifically to a method for producing an α-amino-N-(fluorine atom-containing alkyl) alkanamide compound.

### [Background Art]

2-Amino-N-(2,2,2-trifluoroethyl)acetamide or a salt thereof is a useful compound as an intermediate for pharmaceuticals and agricultural chemicals.

As a method for producing 2-amino-N-(2,2,2-trifluoroethyl)acetamide, for example, Patent Document 1 discloses a method of producing 2-amino-N-(2,2,2-trifluoroethyl)acetamide by chemically reacting 2,2,2-trifluoroethylamine or a salt thereof with an acyl compound represented by formula (A) in the presence of an inorganic base to obtain an N-(2,2,2-trifluoroethyl)acetamide compound represented by formula (B), and then deprotecting the phthalimide moiety. 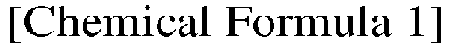 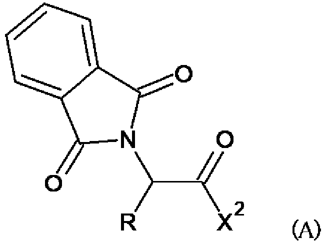 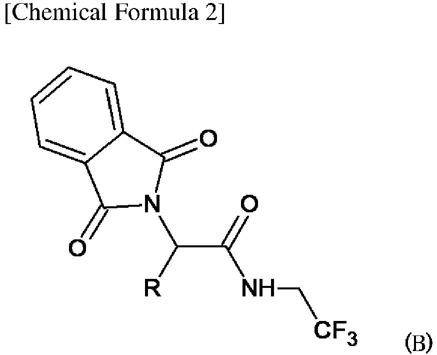 (In formula (A), X² is an alkoxy group, a hydroxy group, a halogen atom, or the like. In formulas (A) and (B), R is a hydrogen atom or a methyl group.)

Patent Document 2 discloses that a compound represented by formula (C) and 2,2,2-trifluoroethylamine hydrochloride are chemically reacted to produce a compound represented by formula (D) (2-amino-N-(2,2,2-trifluoroethyl)propanamide hydrochloride). Patent Document 3 also discloses that 2-amino-2-methyl-N-(2,2,2-trifluoroethyl)propanamide hydrochloride is produced by a similar scheme.

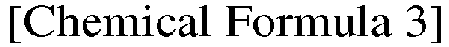

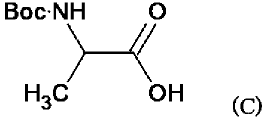

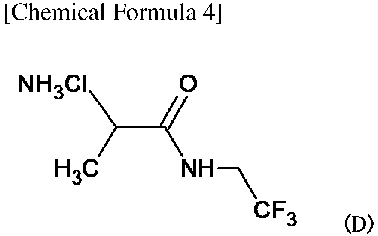

Meanwhile, amino acid N-carboxylic anhydrides are extremely useful intermediates because the amino groups present in the molecules are protected. The acid anhydride group in amino acid N-carboxylic anhydrides is highly active and can react with any nucleophilic unit. For example, it can chemically react with an amino group to obtain an amide group. When amines are reacted with amino acid N-carboxylic anhydrides, they initiate a ring-opening polymerization reaction.

For example, Non-Patent Document 1 discloses that a compound represented by formula (E) is reacted with an amine compound represented by formula (F) to cause a ring-opening polymerization reaction of the compound represented by formula (E), thereby producing a polypeptide represented by formula (G).

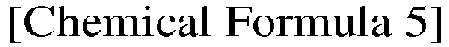

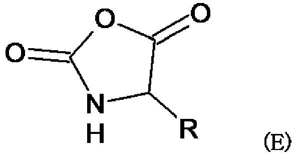

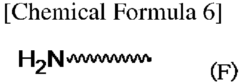

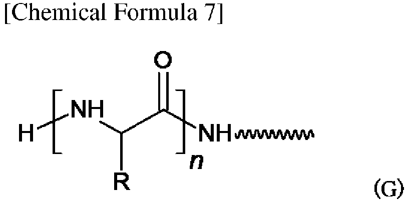

Non-Patent Document 2 discloses that 3-methyl-1,3-oxazolidine-2,5-dione is reacted with 2,2-dimethylpropan-1-amine as an initiator to cause a ring-opening polymerization reaction of 3-methyl-1,3-oxazolidine-2,5-dione, thereby producing a polypeptide having an initiator residue bound to its terminal.

Non-Patent Document 3 discloses that when a compound represented by formula (H1), (H2), or (H3) is allowed to act on 4,4-dimethyl-1,3-oxazolidine-2,5-dione as an initiator, 4,4-dimethyl-1,3-oxazolidine-2,5-dione undergoes a ring-opening polymerization reaction to produce a polypeptide having an initiator residue bound to its terminal.

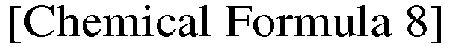

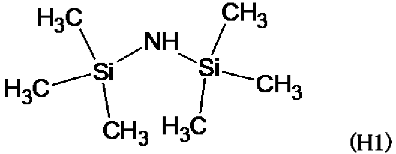

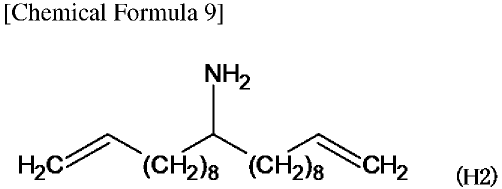

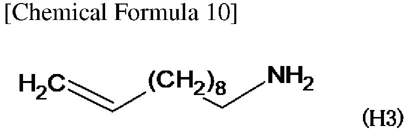

In Non-Patent Document 4, various studies have been conducted to determine whether the polymerization reaction of N-carboxyamino acid anhydrides can be inhibited, and it is described that when N-carboxyamino acid anhydrides are reacted with amines, the reaction is affected by the basicity of the amine, the basicity of the NH of the N-carboxyamino acid anhydride, the basicity of the NH₂ of the peptide produced after the reaction, or the steric hindrance of the substituent of the N-carboxyamino acid anhydride.

### [Citation List]

### [Patent documents]

[Patent Document 1] Japanese Unexamined Patent Application, First Publication No. 2009-173621
[Patent Document 2]: PCT International Publication No. WO 2006/127587 A1
[Patent Document 3] PCT International Publication No. WO 2019/198592 A1

### [Non-patent Documents]

[Non-Patent Document 1] Endo et al. "Development of α-Amino Acid N-carboxylic Anhydride (NCA) Synthesis Method by Phosgene-free Method and its Application to Polypeptide Synthesis", Journal of the Japan Adhesion Society, VOL.52, NO. 11, (2016)333(10)-341(18)
[Non-Patent Document 2] Olga Schafer et al. "Combining Orthogonal Reactive Groups in Block Copolymers for Functional Nanoparticle Synthesis in a Single Step" ACS Macro Lett. 2017, 6, 1140-1145
[Non-Patent Document 3] Jan Freudenberg et al. "Chirality Control of Screw-Sense in Aib-Polymers: Synthesis and Helicity of Amino Acid Functionalized Polymers" ACSMacro Lett. 2020, 9, 686-692
[Non-Patent Document 4] Oya et al. "Acylation Reaction Using N-carboxyamino Acid Anhydride", Organic Synthetic Chemistry, Vol. 29, No. 8 (1971) 751 (15)-759 (23)

### [Summary of Invention]

### [Technical Problem]

An object of the present invention is to provide a method for producing an α-amino-N-(fluorine atom-containing alkyl)alkanamide compound.

### [Solution to Problem]

Based on the reaction mechanism described in Non-Patent Document 1, Non-Patent Document 2, or Non-Patent Document 3, it can be assumed that when a compound represented by formula (1) (hereinafter sometimes referred to as compound (1)) is reacted with a compound represented by formula (2) (hereinafter sometimes referred to as compound (2)), compound (1) will undergo a ring-opening polymerization reaction to produce a polypeptide having a corresponding structure, and compound (3) will not be obtained.

However, in reality, when compound (1) was reacted with compound (2), no polypeptide was produced, and a compound represented by formula (3) (hereinafter sometimes referred to as compound (3)) was obtained. Based on this fact, the present inventors conducted research to achieve the object of the present invention, and as a result, they have completed the present invention, which includes the following aspects.

[1] A method for producing a compound represented by formula (3) and/or a salt thereof, comprising
   chemically reacting a compound represented by formula (1) with at least one selected from the group consisting of a compound represented by formula (2) and a salt thereof. 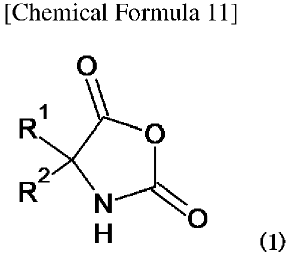
   (In formula (1), R¹ and R² each independently represents a hydrogen atom or a C1-6 alkyl group.) 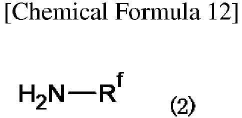
   (In formula (2), R¹ is a fluorine atom-containing C1-6 alkyl group.) 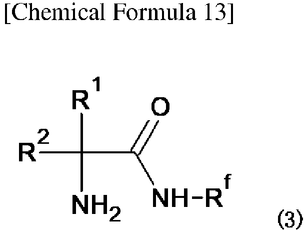
   (In formula (3), R¹ and R² are the same as those in formula (1), and R^{f} is the same as that in formula (2).)
[2] The method according to [1], wherein R¹ and R² in formula (1) and formula (3) are each independently a C1-6 alkyl group.
[3] The method according to [1] or [2], wherein the compound represented by formula (2) is 2,2,2-trifluoroethylamine.

### [Advantageous Effects of Invention]

According to the production method of the present invention, compound (3) can be obtained in high yield. Although the factors contributing to the effectiveness of the present invention are not known, it is believed it is because the nucleophilicity of compound (2) is superior to that of compound (3), or there is steric hindrance due to the fluorine atom in compound (2).

### [Description of Embodiments]

The process for producing compound (3) and/or a salt thereof of the present invention comprises chemically reacting compound (1) with compound (2) and/or a salt thereof.

Compound (1) used in the present invention is represented by formula (1).

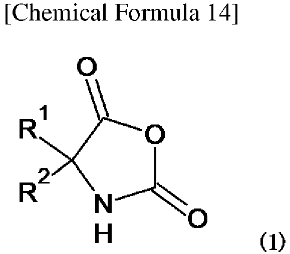

In formula (1), R¹ and R² each independently represents a hydrogen atom or a C1-6 alkyl group.

R¹ and R² for the C1-6 alkyl group may be linear or branched. Examples of the C1-6 alkyl group include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group, a 2,2-dimethylpropyl group, an i-hexyl group and the like.

Compound (1) used in the present invention may be a compound synthesized by oneself using a known method, or may be a commercially available compound synthesized by someone else using some kind of method.

Compound (1) can be obtained, for example, by a method comprising reacting a compound represented by formula (5) (hereinafter sometimes referred to as compound (5)) with phosgene, diphosgene, or triphosgene, a method comprising reacting a compound represented by formula (6) (hereinafter sometimes referred to as compound (6)) with a halogenating reagent to eliminate the halide of R, a method comprising reacting compound (5) with di-tert-butyl tricarbonate, a method comprising reacting compound (5) with a diaryl carbonate, or the like. 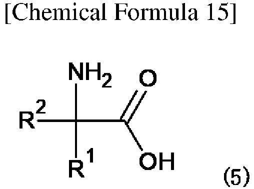 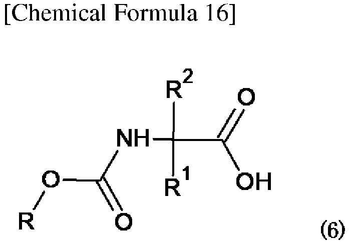 (In formulas (5) and (6), R¹ and R² are the same as those in formula (1). In formula (6), R is a hydrogen atom or an organic group capable of forming a halide.)

Compound (2) used in the present invention is represented by formula (2).

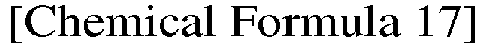

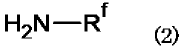

In formula (2), R^{f} is a fluorine atom-containing C1-6 alkyl group.

Examples of the fluorine atom-containing C1-6 alkyl group include a fluoromethyl group, a difluoromethyl group, a trifluoromethyl group, a 1-fluoroethyl group, a 2-fluoroethyl group, a 2,2-difluoroethyl group, a 1,2-difluoroethyl group, a 2,2,2-trifluoroethyl group, a perfluoroethyl group, a 1,2-difluoro-n-propyl group, a 3,3,3-trifluoro-n-propyl group, a 1-fluoro-n-butyl group, a 1-trifluoromethyl-1-methyl-ethyl group, a perfluoro-n-pentyl group, a perfluoro-n-hexyl group and the like.

Examples of the salt of compound (2) include a hydrochloride, a hydrofluoride, a hydrobromide, a nitrate, a sulfonate, a sulfate, a carbonate, an acetate and the like.

Compound (2) and its salt used in the present invention may be a compound synthesized by oneself using a known method, or may be a commercially available compound synthesized by someone else using some kind of method.

In the chemical reaction between compound (1) and compound (2) and/or a salt thereof (hereinafter, sometimes simply referred to as "amidation reaction"), although the amount of compound (2) and/or a salt thereof is not particularly limited, it is preferably 100 to 1,000 parts by mole, more preferably 150 to 300 parts by mole, with respect to 100 parts by mole of compound (1).

The amidation reaction can be carried out in a solvent such as water, an organic solvent, or an ionic liquid. The organic solvent is preferably inert to the chemical reaction. Examples of the organic solvent include alcohols such as methanol, ethanol or the like; ethers such as diethyl ether, diisopropyl ether, diethylene glycol dimethyl ether (product name: diglyme), tetrahydrofuran (abbreviation: THF), dioxane or the like; halogenated hydrocarbons such as dichloromethane, chloroform, 1,2-dichloroethane (abbreviation: DCE) or the like; aliphatic hydrocarbons such as pentane, hexane, cyclohexane, heptane, octane or the like; aromatic hydrocarbons such as toluene, xylene or the like; esters such as methyl acetate, ethyl acetate, propyl acetate, butyl acetate, dimethyl carbonate, diethyl carbonate or the like; ketones such as acetone, anone, methyl isobutyl ketone (abbreviation: MIBK) or the like; and other solvents such as dimethyl sulfoxide (abbreviation: DMSO), dimethylformamide (abbreviation: DMF) or the like. Although the amount of the solvent to be used is not particularly limited, it is preferably 0.5 to 500 parts by weight with respect to 1 part by weight of compound (1).

Although the order of mixing the reaction substrates is not particularly limited, it may be such that, for example, after the solvent is placed in the reaction vessel, compound (2) and/or a salt thereof is added, and then compound (1) is added; alternatively, after the solvent is placed in the reaction vessel, compound (1) is added, and then compound (2) and/or a salt thereof is added. The temperature during mixing is not particularly limited, and may be room temperature. The reaction substrates are preferably mixed under stirring.

The temperature during the amidation reaction can be appropriately selected, and for example, it may be from 0°C to the boiling point of the solvent. The pressure during the amidation reaction is not particularly limited, and for example, it may be normal pressure. The reaction time may be, for example, 0.5 hours to 24 hours.

Compound (3) obtained by the production method of the present invention is represented by formula (3).

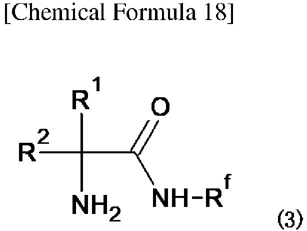

(In formula (3), R¹ and R² are the same as those in formula (1), and R^{f} is the same as that in formula (2).)

Examples of the salt of compound (3) include a hydrochloride, a hydrofluoride, a hydrobromide, a nitrate, a sulfonate, a sulfate, a carbonate, an acetate, and the like.

After the amidation reaction is completed, post-treatment may be carried out. For example, the product may be extracted with an organic solvent, and the resulting organic layer may be dried and concentrated. Furthermore, the product may be purified by recrystallization, chromatography, or the like.

The present invention will be described in more detail below with reference to examples, but the present invention is not limited to the following examples.

### [Example 1] Synthesis of 2-amino-2-methyl-N-(2,2,2-trifluoroethyl)propanamide

10 mL of ethyl acetate was added to 4,4-dimethyloxazolidine-2,5-dione (1.3 g, 10 mmol). 2,2,2-Trifluoroethylamine (1.0 g, 12 mmol) was added dropwise to the resulting solution and reacted at 20°C for 24 hours. The resulting solution was concentrated to dryness to obtain 2-amino-2-methyl-N-(2,2,2-trifluoroethyl)propanamide in a yield of 90%. The NMR of the obtained substance was as follows.

¹H-NMR (CDCl₃/TMS,δ (ppm)): 8.17(s,1H), 3.83-3.92(m,2H), 1.50(s,2H), 1.38(s,6H)

### [Example 2] Synthesis of 2-amino-2-methyl-N-(2,2,2-trifluoroethyl)propanamide

2,2,2-Trifluoroethylamine (51.5 g, 0.61 mol) was added to 100 mL of ethyl acetate. A solution of 4,4-dimethyloxazolidine-2,5-dione (32.7 g, 0.26 mol) dissolved in 150 mL of ethyl acetate was added dropwise to the resulting solution, and the resulting mixture was allowed to react at room temperature for 2 hours. The resulting solution was concentrated to dryness to obtain 46.7 g of 2-amino-2-methyl-N-(2,2,2-trifluoroethyl)propanamide in a yield of 98%. The NMR of the obtained substance was as follows.

¹H-NMR (CDCl₃/TMS,δ (ppm)): 8.17 (s,1H), 3.83-3.92(m,2H), 1.50(s,2H), 1.38(s,6H) M.P. 91-93°C

## Claims

1. A method for producing a compound represented by formula (3) or a salt thereof, comprising
chemically reacting a compound represented by formula (1) with at least one selected from the group consisting of a compound represented by formula (2) and a salt thereof, 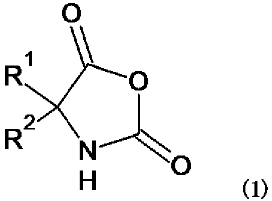
in formula (1), R¹ and R² each independently represents a hydrogen atom or a C1-6 alkyl group, 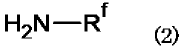
in formula (2), R^{f} is a fluorine atom-containing C1-6 alkyl group, 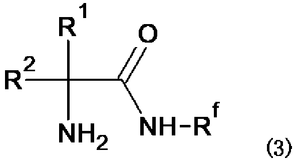
in formula (3), R¹ and R² are the same as those in formula (1), and R^{f} is the same as that in formula (2).

2. The method according to Claim 1, wherein R¹ and R² in formula (1) and formula (3) are each independently a C1-6 alkyl group.

3. The method according to Claim 1 or 2, wherein the compound represented by formula (2) is 2,2,2-trifluoroethylamine.
